# EUROPEAN PATENT APPLICATION

(11) **EP 4 368 986 A1**
(43) Date of publication of application: **15.05.2024**
(21) Application number: 22837386.6
(22) Date of filing: 08.06.2022
(51) Int. Cl.: G01N 29/24

(54) **PHOTOACOUSTIC PHYSICAL PROPERTY MEASURING DEVICE AND MEASURING METHOD**

(30) Priority: 07.07.2021 JP 2021112746
(71) Applicant: HITACHI HIGH-TECH CORPORATION, Tokyo 105-6409 (JP)
(72) Inventor: KOHMU Naohiro, Tokyo 100-8280 (JP); ONO Goichi, Tokyo 100-8280 (JP); KATAGISHI Makoto, Tokyo 105-6409 (JP); DOI Hideaki, Tokyo 105-6409 (JP); SHIRAMIZU Nobuhiro, Tokyo 105-6409 (JP)
(74) Representative: Strehl Schübel-Hopf & Partner
(86) International application number: PCT/JP2022/023041
(87) International publication number: WO 2023/281967

(57) **Abstract**

In a photo-acoustic physical property measuring device, in order to be capable of relatively easily responding to changes in a resonance frequency due to an arrangement state of a measurement object and a state of a gap between a photo-acoustic cell and a measurement object and to enable measurement of a physical property of the object in a relatively short time without lowering detection sensitivity, the photo-acoustic physical property measuring device includes: a light irradiation unit configured to irradiate the object with modulated light; a photo-acoustic cell configured to amplify a photo-acoustic wave generated at the object when the object is irradiated with the modulated light from the light irradiation unit; a sound wave generation unit configured to generate a sound wave inside the photo-acoustic cell; a microphone configured to detect a photo-acoustic wave amplified by the photo-acoustic cell and a sound wave generated inside the photo-acoustic cell from the sound wave generation unit; and a physical property analysis unit configured to analyze the physical property of the object based on the photo-acoustic wave detected by the microphone.

## Description

### Technical Field

The present invention relates to a photo-acoustic physical property measuring device and a measuring method.

### Background Art

In recent years, there has been an increasing need for "non-destructive measurement" capable of inspecting an object without destruction in product inspection. In particular, non-destructive measurement of an organic substance is performed a plurality of times at the time of receiving a raw material of an electric or electronic device or the like, during manufacturing processes, or immediately before shipment. For the non-destructive measurement of an organic substance, photo-acoustic spectroscopy capable of measurement regardless of a state (solid, liquid, gas, powder) of a measurement object is effective.

A principle of a physical property measuring device using the photo-acoustic spectroscopy is as follows. When the measurement object is irradiated with intermittent light from a light source, the measurement object absorbs the light to generate heat, and the heat is converted into kinetic energy with which expansion and contraction locally repeat on a surface. As a result, fluctuation (photo-acoustic wave) occurs in a pressure in a space, and the fluctuation is detected by a microphone. By performing signal processing (physical property analysis) on the detection result, it is possible to evaluate substances and contaminants.

However, since the photo-acoustic wave generated by a photo-acoustic effect is weak, the generated acoustic wave needs to be confined and resonance-amplified in the photo-acoustic cell in order to implement a highly sensitive non-destructive measuring device.

When the photo-acoustic cell has a resonance structure, a weak sound wave can be amplified, and on the other hand, when the resonance frequency at which the photo-acoustic wave resonates in the photo-acoustic cell does not match with a measurement frequency, measurement sensitivity changes or decreases. Further, when a gap is generated between the photo-acoustic cell and the measurement object, an effective size of the photo-acoustic cell changes, and the resonance frequency of the photo-acoustic wave changes.

Therefore, when the measurement is performed without correcting a change in the resonance frequency due to an arrangement state of the measurement object, a state of the gap between the photo-acoustic cell and the measurement object, or the like, the effect of the resonance amplification is not obtained, and the detection sensitivity decreases. Here, a measuring method of searching for the resonance frequency of the photo-acoustic cell before the measurement and performing the measurement using the search result is important.

Regarding the setting of the resonance frequency of the photo-acoustic cell, for example, the technique of PTL 1 is reported. In a component concentration measurement device described in PTL 1, a plurality of laser light sources having different wavelengths are prepared, and an acoustic mode is estimated from a change amount in the light intensity of the laser light sources to adjust a length of the photo-acoustic cell, and thereafter, the measurement is performed to compensate for acoustic mode dependence of concentration sensitivity of a component to be measured.

### Citation List

### Patent Literature

PTL 1: JP2018-171178A

### Summary of Invention

### Technical Problem

In the method described in PTL 1, since a physical length of the acoustic cell is changed, there is a possibility that compensation cannot be performed when the resonance frequency greatly changes in the above-described problem. Further, since the length of the photo-acoustic cell is adjusted, it takes time to specify the resonance frequency, and when a large number of locations are sequentially measured, throughput may be deteriorated.

The invention solves the above-described problems of the related art, and provides a photo-acoustic physical property measuring device and a measuring method capable of relatively easily responding to changes in a resonance frequency due to an arrangement state of a measurement object and a state of a gap between the photo-acoustic cell and the measurement object and capable of measuring a physical property of the object in a relatively short time without lowering the detection sensitivity.

### Solution to Problem

In order to solve the above-described problems, in the invention, a photo-acoustic physical property measuring device, which measures a property of an object by irradiating the object with light to detect a photo-acoustic signal, includes: a light irradiation unit configured to irradiate the object with modulated light; a photo-acoustic cell configured to amplify a photo-acoustic wave generated at the object when the object is irradiated with the modulated light from the light irradiation unit; a sound wave generation unit configured to generate a sound wave inside the photo-acoustic cell; a microphone configured to detect a photo-acoustic wave amplified by the photo-acoustic cell and a sound wave generated inside the photo-acoustic cell from the sound wave generation unit; and a physical property analysis unit configured to analyze the physical property of the object based on the photo-acoustic wave detected by the microphone.

In order to solve the above-described problems, in the invention, a photo-acoustic physical property measuring method includes: irradiating an object with light that is emitted from a light irradiation unit and is modulated at a predetermined frequency; detecting, by a microphone, a photo-acoustic signal that is generated from a sample irradiated with the light modulated at the predetermined frequency and resonates inside the photo-acoustic cell; and analyzing a physical property of the sample using the photo-acoustic signal detected by the microphone. The predetermined frequency of the light emitted from the light irradiation unit to the object is set based on a peak frequency searched for by a peak frequency search unit, and the peak frequency is obtained by generating a sound wave inside the photo-acoustic cell from a speaker; receiving, by the microphone, a sound generated inside the photo-acoustic cell by the sound wave, and searching for, by the peak frequency search unit, a peak frequency of the sound received by the microphone.

Further, in order to solve the above-described problems, in the invention, a photo-acoustic physical property measuring method includes: irradiating an object with light that is emitted from a light irradiation unit and is modulated at a predetermined frequency; detecting, by a microphone, a photo-acoustic signal that is generated from a sample irradiated with the light modulated at the predetermined frequency and resonates inside the photo-acoustic cell; and analyzing a physical property of the sample using the photo-acoustic signal detected by the microphone. The predetermined frequency of the light emitted from the light irradiation unit to the object is set based on a peak frequency searched for by a peak frequency search unit, and the peak frequency is obtained by sequentially irradiating the sample with light having a different frequency to be modulated from the light irradiation unit, obtaining and storing a frequency characteristic of a photo-acoustic signal that resonates inside the photo-acoustic cell from a signal detected by the microphone, generating a sound wave inside the photo-acoustic cell from a speaker; receiving, by the microphone, a photo-acoustic signal generated inside the photo-acoustic cell by the sound wave, searching for, by the peak frequency search unit, a peak frequency from a signal previously obtained by subtracting a component of the obtained and stored frequency characteristic from the photo-acoustic signal received by the microphone.

### Advantageous Effects of Invention

According to the invention, it is possible to relatively easily respond to changes in the resonance frequency due to the arrangement state of the measurement object and the state of the gap between the photo-acoustic cell and the measurement object, and thus, a physical property of the object can be measured in a relatively short time without lowering the detection sensitivity.

### Brief Description of Drawings

[FIG. 1] FIG. 1 is a block diagram showing a schematic configuration of a photo-acoustic physical property measuring device according to a first embodiment of the invention.
[FIG. 2] FIG. 2 is a flowchart showing a procedure of a photo-acoustic physical property measuring method according to the first embodiment of the invention.
[FIG. 3] FIG. 3 is a graph showing a relationship between a frequency and a sound wave intensity showing a principle of the photo-acoustic physical property measuring method according to the first embodiment of the invention.
[FIG. 4] FIG. 4 is a graph showing frequency characteristics of sound waves generated from a speaker according to the first embodiment of the invention.
[FIG. 5] FIG. 5 is a block diagram showing a schematic configuration of a photo-acoustic physical property measuring device according to a second embodiment of the invention.
[FIG. 6] FIG. 6 is a flowchart showing a procedure of a photo-acoustic physical property measuring method according to the second embodiment of the invention.
[FIG. 7] FIG. 7 is a graph showing frequency characteristics of sound waves generated from a speaker according to the second embodiment of the invention.
[FIG. 8] FIG. 8 is a block diagram showing a schematic configuration of a photo-acoustic physical property measuring device according to a third embodiment of the invention.
[FIG. 9] FIG. 9 is a graph showing an example of a frequency characteristic of a photo-acoustic signal detected by a frequency search unit.
[FIG. 10] FIG. 10 is a graph showing another example of the frequency characteristic of the photo-acoustic signal detected by the frequency search unit.
[FIG. 11] FIG. 11 is a block diagram showing a schematic configuration of a photo-acoustic physical property measuring device according to a fourth embodiment of the invention.
[FIG. 12] FIG. 12 is a block diagram showing a schematic configuration of a photo-acoustic physical property measuring device according to a fifth embodiment of the invention.
[FIG. 13] FIG. 13 is a block diagram showing a schematic configuration of a photo-acoustic physical property measuring device according to a sixth embodiment of the invention.
[FIG. 14] FIG. 14 is a block diagram showing a schematic configuration of a photo-acoustic physical property measuring device according to a seventh embodiment of the invention.
[FIG. 15] FIG. 15 is a flowchart showing a procedure of a photo-acoustic physical property measuring method according to the seventh embodiment of the invention.
[FIG. 16] FIG. 16 is a block diagram showing a schematic configuration of a photo-acoustic physical property measuring device according to an eighth embodiment of the invention.

### Description of Embodiments

In the invention, before measurement, a broadband sound wave is generated from a speaker disposed in a photo-acoustic cell, and detection and signal processing are performed on the sound wave by a microphone to search for a resonance frequency, a speed of frequency calibration is increased, and high sensitivity of the measurement is implemented.

Further, in the invention, a sound source such as a speaker is attached to the photo-acoustic cell, and a resonance frequency between the photo-acoustic cell and a measurement object is obtained by detecting a frequency characteristic of the sound wave oscillated from the sound source, and thus, time required to obtain the resonance frequency can be shortened as compared with that in the related art, and throughput of physical property measurement using photo-acoustic is improved.

Further, the resonance frequency is obtained by detecting the frequency characteristic of the broadband sound wave oscillated from the sound source attached to the photo-acoustic cell, and thus, even when a variation of the resonance frequency is large, a peak frequency can be detected, and highly sensitive non-destructive measurement can be implemented.

According to the invention, a photo-acoustic physical property measuring device includes a light source, a drive circuit, a photo-acoustic cell, a microphone, a high-sensitivity amplifier circuit, a physical property analysis unit, a measurement area for storing a sample, a signal generation circuit, a speaker, and a peak frequency search unit. Before the measurement, a broadband sound wave is generated from the signal generation circuit and the speaker, the peak frequency of the signal detected by the microphone is calculated by the peak frequency search unit, the light source is driven using the frequency calculated by the peak frequency search unit to irradiate the sample with light, and the measurement is performed by the microphone, the high-sensitivity amplifier circuit, and the physical property analysis unit.

The invention also provides a photo-acoustic physical property measuring device including a light source, a drive circuit, a photo-acoustic cell, a microphone, a high-sensitivity amplifier circuit, a physical property analysis unit, a signal generation circuit, a speaker, and a peak frequency search unit. Before the measurement, a broadband sound wave is generated from the signal generation circuit and the speaker, the peak frequency of the signal detected by the microphone is calculated by the peak frequency search unit, the light source is driven using the frequency to irradiate the sample with light, the measurement is performed by the microphone, the high-sensitivity amplifier circuit, and the physical property analysis unit, and the variation of the resonance frequency is corrected. Therefore, the variation of the resonance frequency of the photo-acoustic cell due to an arrangement and shape of the sample is prevented, the measurement sensitivity is prevented from deteriorating, and highly sensitive photo-acoustic measurement can be implemented.

Hereinafter, embodiments according to the invention will be described in detail with reference to drawings. In all the drawings showing the embodiments, components having the same function are denoted by the same reference numerals, and the repetitive description thereof will be omitted in principle.

However, the invention should not be construed as being limited to the description of the embodiments described below. Those skilled in the art could easily understand that the specific configuration of the invention can be changed without departing from a spirit or a gist of the invention.

### Embodiment 1

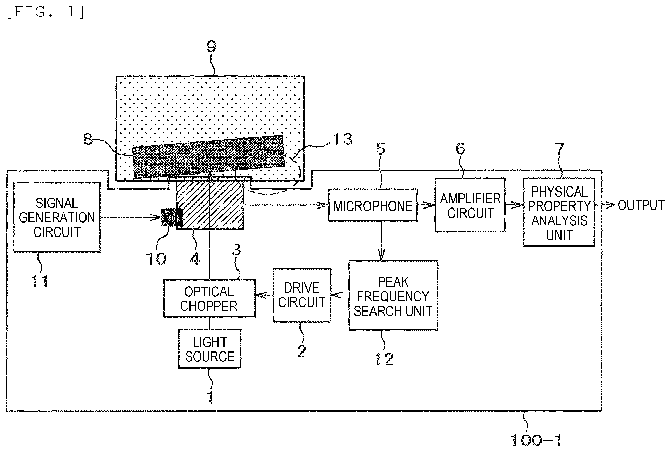

Embodiment 1 according to the invention will be described with reference to FIGS. 1 to 4. FIG. 1 shows a configuration of a photo-acoustic physical property measuring device 100-1 according to the present embodiment. The photo-acoustic physical property measuring device 100-1 according to the present embodiment includes a light source 1, a drive circuit 2, an optical chopper 3, a photo-acoustic cell 4, a microphone 5, an amplifier circuit 6, a physical property analysis unit 7, a speaker 10, a signal generation circuit 11, and a peak frequency search unit 12. In the example shown in FIG. 1, the photo-acoustic cell 4 is brought close to a sample 8 stored in a measurement area 9, and physical properties of the sample 8 are measured.

As the light source 1, a laser or a white light source is used. As the speaker 10, a MEMS speaker or an electret speaker is used.

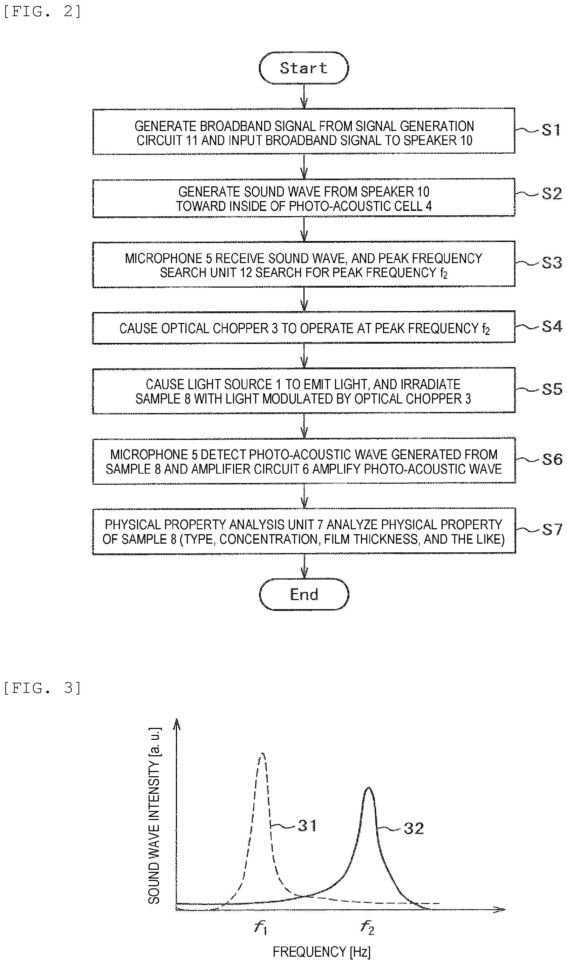

As in the example shown in FIG. 1, the photo-acoustic cell 4 and the sample 8 may be inclined instead of being in contact with each other, and a gap 13 as indicated by a dotted line may be formed. When the photo-acoustic cell 4 and the sample 8 are in the state shown in FIG. 1, as shown in FIG. 3, a resonance frequency f2: 32 between the photo-acoustic cell 4 and the sample 8 deviates from a resonance frequency f1: 31 in an ideal state in which the photo-acoustic cell 4 and the sample 8 are in contact with each other.

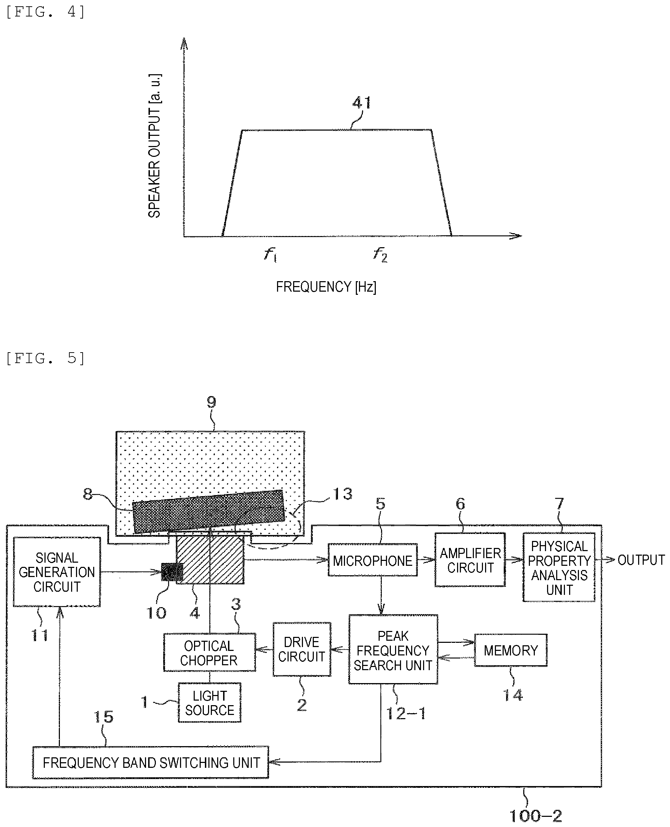

Therefore, in the present embodiment, in consideration of the deviation of the resonance frequency as shown in FIG. 3, calibration measurement for obtaining the resonance frequency is performed using a sound wave 41 having a frequency characteristic of a relatively wide frequency band as shown in FIG. 4, and then the measurement is performed at the calibrated frequency.

A procedure for measuring the physical properties of the sample 8 using the photo-acoustic physical property measuring device 100-1 having the configuration shown in FIG. 1 will be described with reference to a flowchart of FIG. 2.

First, in the state in which the photo-acoustic cell 4 is in contact with the sample 8, a broadband signal is generated from the signal generation circuit 11 and is input to the speaker 10 (S1). The speaker 10 to which the broadband signal is input generates a broadband sound wave having a wide bandwidth as shown in FIG. 4, for example, toward the inside of the photo-acoustic cell 4 (S2). The broadband sound wave generated from the speaker 10 toward the photo-acoustic cell 4 is received by the microphone 5, and the signal received by the microphone 5 is processed by the peak frequency search unit 12 to search for the peak frequency f2 (S3). The processing of S1 to S3 is the calibration measurement.

Next, in the measurement, the drive circuit 2 causes the optical chopper 3 to operate at the peak frequency f2 (S4), the light source 1 is caused to emit light, and the sample 8 is irradiated with the light that is modulated by the optical chopper 3 and passes through the photo-acoustic cell 4 (S5).

A photo-acoustic wave generated from the sample irradiated with the modulated light is detected by the microphone 5 and amplified by the amplifier circuit 6 (S6).

The amplified signal is input to the physical property analysis unit 7. The physical property analysis unit 7 searches for a frequency at which a signal intensity is maximized by signal processing such as Fourier transform, and calculates the peak frequency f1. By comparing and analyzing signal information at the obtained peak frequency f1 with data stored in a database, physical properties according to a purpose of the measurement such as a type, concentration, and film thickness of the sample 8 are analyzed (S7) and output.

According to the present embodiment, the sound wave generated from the speaker 10 is detected by the microphone 5, the peak frequency is obtained by the peak frequency search unit 12, and a drive frequency of the optical chopper 3 is corrected, so that a variation of the resonance frequency of the photo-acoustic cell 4 caused by deviation of a positional relationship of the sample 8 with respect to the photo-acoustic cell 4 can be calibrated, and thus, it is possible to perform non-destructive measurement using high-sensitivity photo-acoustic spectroscopy.

### Embodiment 2

Embodiment 2 according to the invention will be described with reference to FIGS. 5 to 7. FIG. 5 shows a configuration of a photo-acoustic physical property measuring device 100-2 according to the present embodiment. The configuration of the photo-acoustic physical property measuring device 100-2 according to the present embodiment is different from the configuration of the photo-acoustic physical property measuring device 100-1 described in FIG. 1 in Embodiment 1 in that a memory 14 and a frequency band switching unit 15 are added.

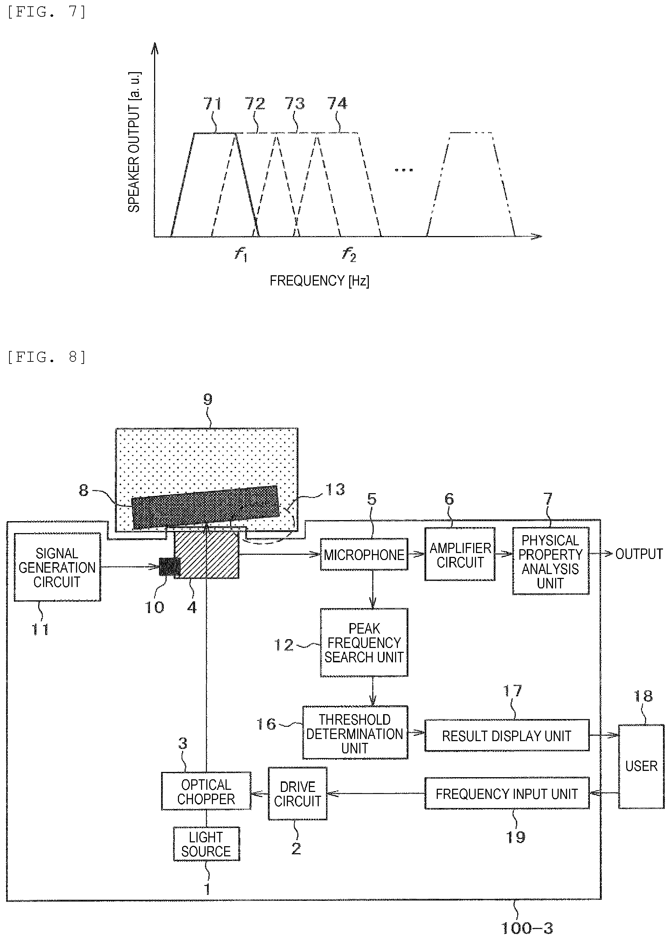

In the present embodiment, as shown in FIG. 7, a sound wave having a frequency characteristic with a relatively small bandwidth as compared with the case of FIG. 4 in Embodiment 1 is generated from the speaker 10. In the present embodiment, the frequency band is divided by the frequency band switching unit 15, the signal generation circuit 11 is driven, and sounds of frequency bands 71, 72, 73, 74, and the like are sequentially switched and generated from the speaker 10.

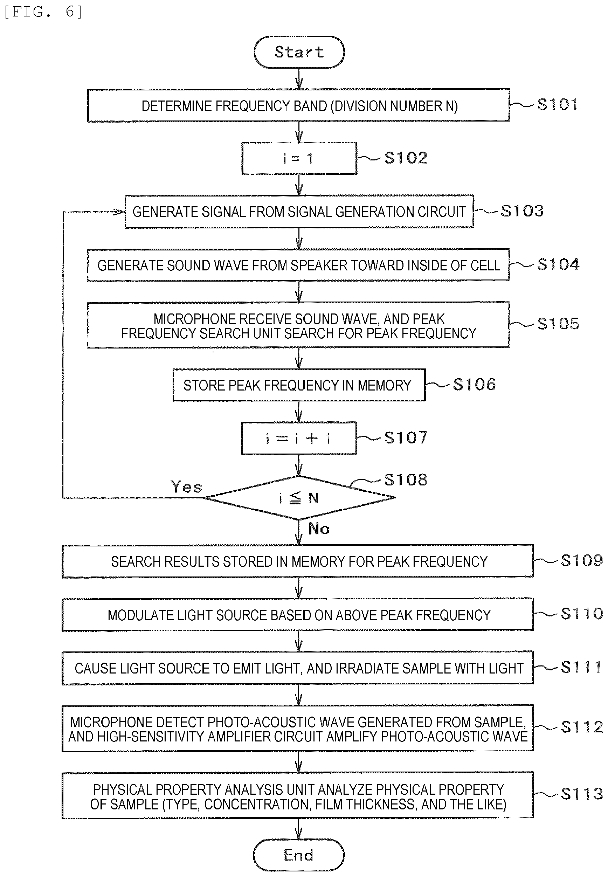

A procedure for measuring the physical properties of the sample 8 using the photo-acoustic physical property measuring device 100-2 having the configuration shown in FIG. 5 will be described with reference to a flowchart of FIG. 6.

First, the frequency band to be divided is determined by the frequency band switching unit 15 (S101). In the example of FIG. 6, a division number is N. Next, i is set to be 1 (S102), and a signal in a first frequency band is transmitted from the signal generation circuit 11 to the speaker 10 in accordance with a command from the frequency band switching unit 15 (S103) . The speaker 10 receiving the signal from the signal generation circuit 11 generates a sound wave in the first frequency band toward the inside of the photo-acoustic cell 4 (S104).

Next, the sound wave generated by the speaker 10 is received by the microphone 5, the received signal from the microphone 5 is received by a peak frequency search unit 12-1, the peak frequency is searched for (S105), and the peak frequency is stored in the memory 14 (S106).

Next, 1 is added to i (S107), and the processing from S103 to S105 is repeatedly executed until i reaches the division number N set in S101 (S108).

When i becomes N (No in S108), the peak frequency search unit 12-1 searches signals of peak frequencies in the respective frequency bands stored in the memory 14 for a frequency peak having a highest signal level (S109). Next, the drive circuit 2 controls the optical chopper 3 based on the searched peak frequency to modulate light emitted from the light source 1 (S110), and the sample 8 is irradiated with the modulated light (S111).

A photo-acoustic wave generated from the sample irradiated with the modulated light is detected by the microphone 5 and amplified by the amplifier circuit 6 (S112).

The amplified signal is input to the physical property analysis unit 7. The physical property analysis unit 7 searches for a frequency at which a signal intensity is maximized by signal processing such as Fourier transform, and calculates the peak frequency f1. By comparing and analyzing signal information at the obtained peak frequency f1 with data stored in a database, physical properties according to a purpose of the measurement such as a type, concentration, and film thickness of the sample 8 are analyzed (S113) and output.

According to the present embodiment, in addition to obtaining the same effect as in the case of Embodiment 1, by dividing the frequency band of the sound wave generated from the speaker 10, it is possible to reduce a total amount of signals input to the microphone 5 and the amplifier circuit 6 for each frequency band and prevent accuracy deterioration of the peak search due to a dynamic range shortage of the microphone 5 and the amplifier circuit 6.

### Embodiment 3

Next, Embodiment 3 will be described with reference to FIGS. 8 to 10.

FIG. 8 shows a configuration of a photo-acoustic physical property measuring device 100-3 according to the present embodiment. The configuration of the photo-acoustic physical property measuring device 100-3 according to the present embodiment is different from the configuration of the photo-acoustic physical property measuring device 100-1 described in FIG. 1 in Embodiment 1 in that a threshold determination unit 16, a result display unit 17, and a frequency input unit 19 are added.

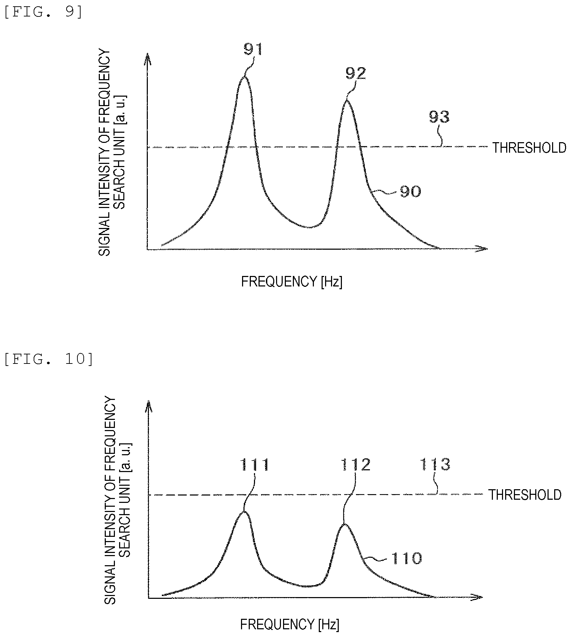

In the Embodiments 1 and 2, the case in which only one peak signal is detected by the peak frequency search unit 12 for each frequency band is described, but as shown in FIG. 9, a signal waveform 90 having a plurality of peaks 91 and 92 each having a level higher than a preset threshold 93 may be detected, or as shown in FIG. 10, a signal waveform 110 in which levels of peaks 111 and 112 are lower than a preset threshold 113 may be detected.

In the present embodiment, in response to the case in which a plurality of peaks are detected as described above, the peak frequency obtained by the peak frequency search unit 12 is compared with a threshold set in advance by the threshold determination unit 16 and determined, and a result is displayed on the result display unit 17 to be notified of to a user 18. The user 18 inputs the frequency for driving the optical chopper 3 to the frequency input unit 19 or corrects the positional relationship between the photo-acoustic cell 4 and the sample 8 based on the displayed result.

That is, a relationship between the peak frequency displayed on the result display unit 17 and the threshold is as follows as shown in FIG. 9, and when the levels of the peaks 91 and 92 are higher than the threshold 93, the frequency for driving the optical chopper 3 is input to the frequency input unit 19 based on the displayed result. In this case, the drive circuit 2 drives the optical chopper 3 based on the frequency received from the frequency input unit 19, and analyzes the physical properties of the sample 8 in the procedure described in Embodiment 1 or 2.

On the other hand, a relationship between the peak frequency displayed on the result display unit 17 and the threshold is as follows as shown in FIG. 10, when both the peaks 111 and 112 are lower than the threshold 113 as shown in FIG. 10, the user 18 suspends the measurement once, corrects the positional relationship between the photo-acoustic cell 4 and the sample 8, and restarts the measurement.

According to the present embodiment, by notifying the user of the peak frequency search result, it is possible to stably perform highly sensitive measurement by selecting the frequency for controlling the optical chopper 3 when the peak value is higher than the threshold value even when a plurality of peaks exist in the detection waveform. On the other hand, when the peak value is lower than the threshold, the positional relationship between the photo-acoustic cell 4 and the sample 8 is corrected, so that the highly sensitive measurement can be reliably performed.

### Embodiment 4

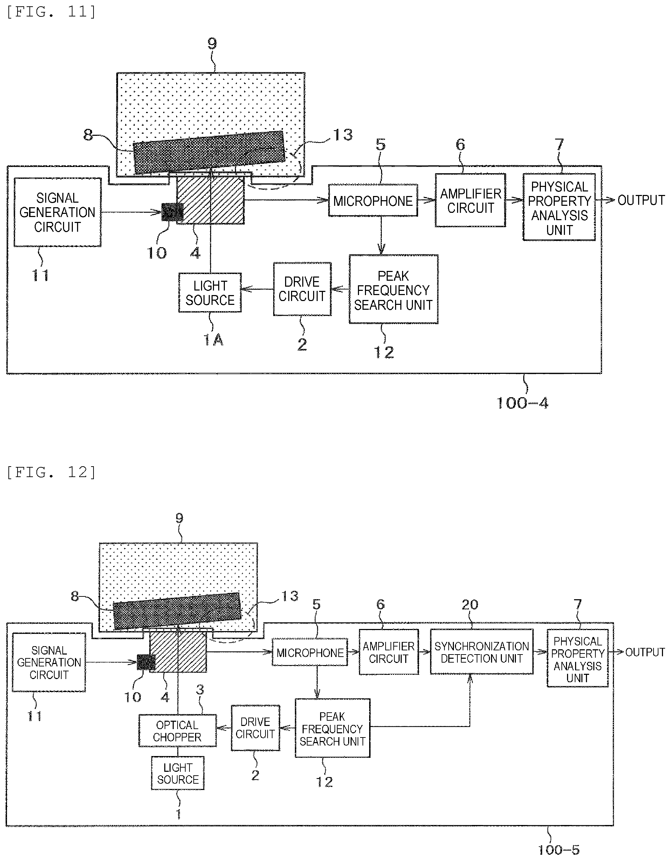

Embodiment 4 according to the invention will be described with reference to FIG. 11. A configuration of a photo-acoustic physical property measuring device 100-4 according to the present embodiment shown in FIG. 11 is different from the configuration of the photo-acoustic physical property measuring device 100-1 shown in FIG. 1 described in Embodiment 1 in that the optical chopper 3 is not provided in the configuration according to the present embodiment shown in FIG. 11.

That is, in the photo-acoustic physical property measuring device 100-4 according to the present embodiment, the drive circuit 2 directly controls a light source 1A based on the peak frequency obtained by the peak frequency search unit 12 from the sound wave signal detected by the microphone 5.

Therefore, in the present embodiment, a light source capable of controlling a modulation frequency, such as a semiconductor laser, is used as the light source 1A.

A procedure for measuring the physical properties of the sample 8 using the photo-acoustic physical property measuring device 100-4 according to the present embodiment is basically the same as that described using the flowchart of FIG. 2 in Embodiment 1, but S4 and S5 are integrated, and the light source 1A emits light at the peak frequency f1 to irradiate the sample 8 with the light.

Although the present embodiment is described with respect to the difference from the configuration of the photo-acoustic physical property measuring device 100-1 described in Embodiment 1, the present embodiment can be applied to the configuration according to Embodiment 2 or Embodiment 3 by removing the optical chopper 3 in the configuration of the photo-acoustic physical property measuring device 100-2 described in Embodiment 2 or the configuration of the photo-acoustic physical property measuring device 100-3 described in Embodiment 3 and replacing the light source 1 with the light source 1A.

According to the present embodiment, in addition to the effects described in Embodiments 1 to 3, since the optical chopper 3 is not used, a size of the device can be reduced accordingly.

### Embodiment 5

Embodiment 5 according to the invention will be described with reference to FIG. 12. A configuration of a photo-acoustic physical property measuring device 100-5 according to the present embodiment shown in FIG. 12 is different from the configuration of the photo-acoustic physical property measuring device 100-1 shown in FIG. 1 described in Embodiment 1 in that the configuration according to the present embodiment shown in FIG. 12 includes a synchronization detection unit 20 coupled to the amplifier circuit 6 and the peak frequency search unit 12.

That is, in the photo-acoustic physical property measuring device 100-5 according to the present embodiment, after the sound wave signal detected by the microphone 5 is amplified by the amplifier circuit 6, a signal synchronized with the frequency of the peak signal detected by the peak frequency search unit 12 is detected in the synchronization detection unit 20, and the signal detected in synchronization is sent to the physical property analysis unit 7.

In a procedure for measuring the physical properties using the photo-acoustic physical property measuring device 100-5 according to the present embodiment, a step of detecting a signal synchronized with the peak frequency f2 from the signals amplified by the amplifier circuit 6 is added between the steps S6 and S7 in the flowchart shown in FIG. 2 in Embodiment 1.

The configuration described in the present embodiment can also be applied to the configurations of Embodiment 2, Embodiment 3, and Embodiment 4 by adding the synchronization detection unit 20 to the configurations of the photo-acoustic physical property measuring device 100-2 described in Embodiment 2, the photo-acoustic physical property measuring device 100-3 described in Embodiment 3, and the photo-acoustic physical property measuring device 100-4 described in Embodiment 4.

According to the present embodiment, in addition to the effects described in Embodiments 1 to 4, it is possible to implement high detection sensitivity.

### Embodiment 6

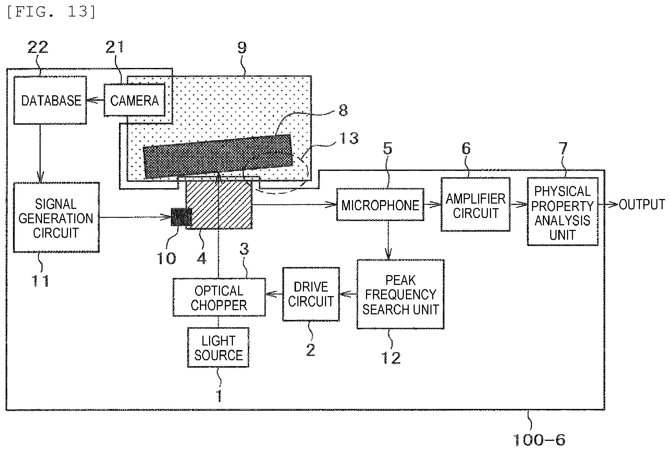

Embodiment 6 according to the invention will be described with reference to FIG. 13. A configuration of a photo-acoustic physical property measuring device 100-6 according to the present embodiment shown in FIG. 13 is different from the configuration of the photo-acoustic physical property measuring device 100-1 shown in FIG. 1 described in Embodiment 1 in that the configuration according to the present embodiment shown in FIG. 13 includes a camera 21 and a database 22. In the database 22, data of the resonance frequency corresponding to an arrangement of the sample 8 (the positional relationship between the photo-acoustic cell 4 and the sample 8) and a material of the sample 8 is recorded.

In the photo-acoustic physical property measuring device 100-6 according to the present embodiment, a shape and the arrangement of the sample 8 are measured by the camera 21, and the measured result is compared with the database 22 to determine a band of the signal frequency generated from the signal generation circuit 11. Accordingly, as compared with the case in which the camera 21 and the database 22 are not used as described in Embodiments 1 to 5, it is possible to narrow the band of the signal frequency generated from the signal generation circuit 11, and it is possible to prevent the accuracy deterioration of the peak search due to the dynamic range shortage of the microphone 5 and the amplifier circuit 6.

A procedure for measuring the physical properties using the photo-acoustic physical property measuring device 100-6 according to the present embodiment is basically the same as the flowchart shown in FIG. 2 in Embodiment 1, but is different in that a step of measuring the shape and the arrangement of the sample 8 by the camera 21 and comparing the measurement result with the database 22 to determine the band of the signal frequency generated from the signal generation circuit 11 is added before S1, and in step S1, the signal of the frequency band determined by referring to the database 22 is input to the speaker 10 from the signal generation circuit 11.

According to the present embodiment, in addition to the effects described in Embodiments 1 to 5, it is possible to prevent the accuracy deterioration of the peak search due to the dynamic range shortage of the microphone 5 and the amplifier circuit 6 and to implement the high detection sensitivity.

### Embodiment 7

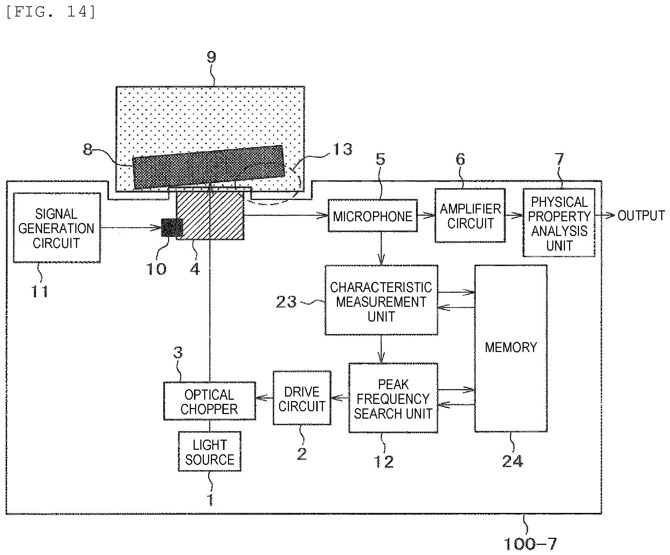

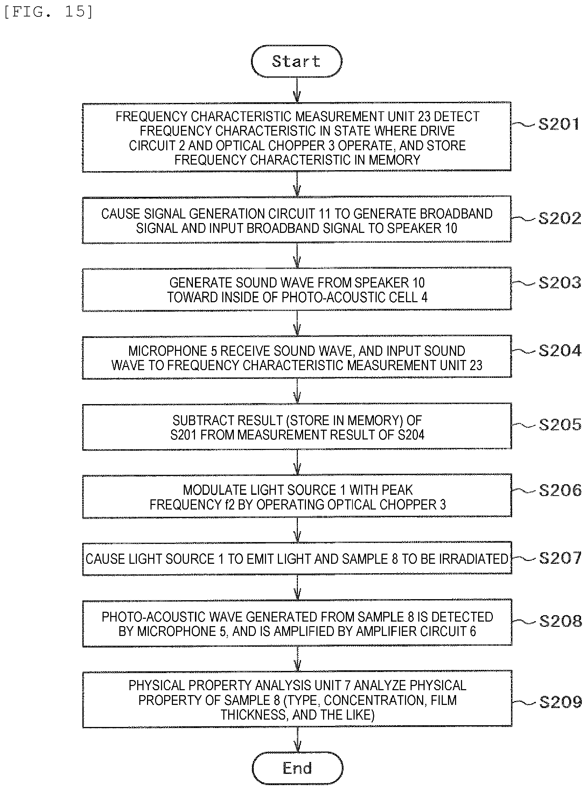

Embodiment 7 according to the invention will be described with reference to FIGS. 14 and 15. When an acoustic wave is detected by the photo-acoustic cell 4, the acoustic wave is affected by ambient vibration or noise, and is added to an output signal of the photo-acoustic cell 4 as a noise component, resulting in deterioration of detection accuracy.

In the present embodiment, the noise component added to the output signal of the photo-acoustic cell 4 is measured and stored in advance, and the noise component measured in advance is subtracted from the detection signal obtained by detecting the photo-acoustic wave from the sample. Accordingly, the dynamic range shortage of the microphone 5 and the amplifier circuit 6 are prevented by expanding a range of a frequency region searched by the peak frequency search unit 12.

FIG. 14 shows a configuration of a photo-acoustic physical property measuring device 100-7 according to the present embodiment. The configuration of the photo-acoustic physical property measuring device 100-7 according to the present embodiment is different from the configuration of the photo-acoustic physical property measuring device 100-1 described in FIG. 1 in Embodiment 1 in that a frequency characteristic measurement unit 23 and the memory 14 are disposed between the microphone 5 and the peak frequency search unit 12.

In the configuration of the photo-acoustic physical property measuring device 100-7, an acoustic wave generated in the photo-acoustic cell 4 by irradiating the sample 8 with intermittent light of a frequency f0 from a light source 1 side in a state in which the broadband sound wave is not emitted from the speaker 10 becomes a noise component with respect to an acoustic wave detected in a state in which the broadband sound wave is emitted from the speaker 10.

In the photo-acoustic physical property measuring device 100-7 according to the present embodiment, first, the optical chopper 3 is driven while changing the drive frequency by the drive circuit 2 in a state in which light is emitted from the light source 1, the sample 8 is irradiated with frequency-modulated light, a photo-acoustic wave is generated inside the photo-acoustic cell 4 and detected by the microphone 5, a frequency characteristic of the output signal from the microphone 5 is measured by the frequency characteristic measurement unit 23, and the frequency characteristic is stored in the memory 14. Next, a signal waveform is created by subtracting a frequency characteristic signal component stored in the memory 14 from the signal waveform output from the microphone 5 that detects the acoustic wave generated in the photo-acoustic cell 4 in a state in which the broadband sound wave is emitted from the speaker 10, and the signal waveform is input to the peak frequency search unit 12 to search for the peak frequency.

A procedure for measuring the physical properties of the sample 8 using the photo-acoustic physical property measuring device 100-7 having the configuration shown in FIG. 14 will be described with reference to a flowchart of FIG. 15.

First, in a state in which the photo-acoustic cell 4 is in contact with the sample 8, the optical chopper 3 is operated while changing the drive frequency by the drive circuit 2, and light emitted from the light source 1 and transmitted through the optical chopper 3 is emitted to the sample 8 via the photo-acoustic cell 4. In this state, the acoustic wave generated in the photo-acoustic cell 4 is detected by the microphone 5, the output signal from the microphone 5 is input to the frequency characteristic measurement unit 23, the frequency characteristic of the acoustic wave detected by the microphone 5 is measured, and the frequency characteristic is input to the memory 14 (S201).

Next, in the state in which the photo-acoustic cell 4 is in contact with the sample 8, a broadband signal is generated from the signal generation circuit 11 and is input to the speaker 10 (S202) . The speaker 10 to which the broadband signal is input generates a broadband sound wave having a wide bandwidth as described with reference to FIG. 4 in Embodiment 1, for example, toward the inside of the photo-acoustic cell 4 (S203). The broadband sound wave generated from the speaker 10 toward the photo-acoustic cell 4 is received by the microphone 5, and the signal received by the microphone 5 is input to the frequency characteristic measurement unit 23 (S204).

The frequency characteristic measurement unit 23 subtracts the result received by the microphone 5 without generating the sound wave from the speaker measured in step S201 and stored in the memory 14 from the result received by the microphone 5 when the broadband sound wave is generated from the speaker measured in step S204, and removes the noise component from the result measured in step S204 (S205).

Next, in the peak frequency search unit 12, the peak frequency f2 as described with reference to FIG. 3 in Embodiment 1 is searched for from the signal from which the noise component is removed in S205, the light emitted from the light source 1 is modulated with the frequency f2 by operating the optical chopper 3 with the peak frequency f2 obtained by the search (S206), and the sample 8 inside the measurement area 9 is irradiated with the light via the photo-acoustic cell 4 (S207).

Next, a photo-acoustic wave generated from the sample 8 irradiated with the light modulated at the frequency f2 is detected by the microphone 5 and amplified by the amplifier circuit 6 (S208).

The amplified signal is input to the physical property analysis unit 7, and the physical property analysis unit 7 searches for a frequency at which a signal intensity is maximized by signal processing such as Fourier transform, and calculates the peak frequency f1. By comparing and analyzing signal information at the obtained peak frequency f1 with data stored in a database, physical properties according to a purpose of the measurement such as a type, concentration, and film thickness of the sample 8 are analyzed (S209) and output.

According to the present embodiment, the sound wave generated from the speaker 10 is detected by the microphone 5, the peak frequency is obtained by the peak frequency search unit 12, and a drive frequency of the optical chopper 3 is corrected, so that a variation of the resonance frequency of the photo-acoustic cell 4 caused by deviation of the positional relationship of the sample 8 with respect to the photo-acoustic cell 4 can be calibrated using the signal from which a background signal is removed, and thus, it is possible to prevent the dynamic range shortage of the microphone 5 and the amplifier circuit 6 when the physical properties of the sample 8 are measured, and it is possible to perform the non-destructive measurement using the high-sensitivity photo-acoustic spectroscopy.

### Embodiment 8

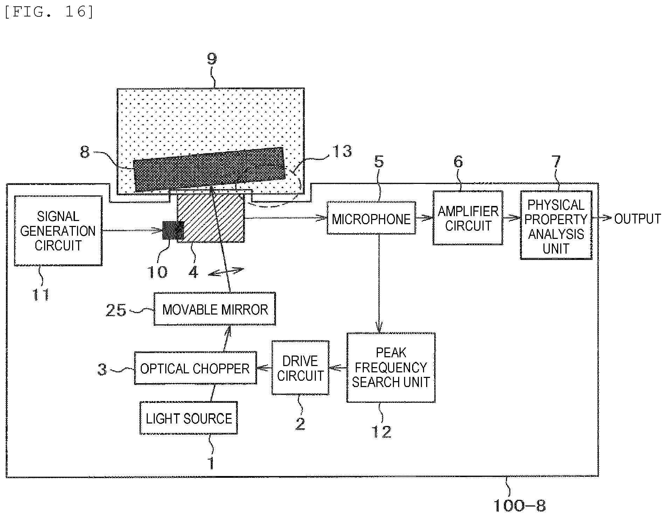

Embodiment 8 according to the invention will be described with reference to FIG. 16. The present embodiment is different from the configuration of Embodiment 1 in that a movable mirror 25 is provided between the optical chopper 3 and the photo-acoustic cell 4, with respect to the configuration described with reference to FIG. 1 in Embodiment 1. In the present embodiment, with such a configuration, a position at which the sample 8 is irradiated with light through the photo-acoustic cell 4 can be displaced without relatively moving the positions of the photo-acoustic cell 4 and the sample 8. Accordingly, in a state in which the position of the photo-acoustic cell 4 is fixed to the measurement area 9 in which the sample 8 is stored, a plurality of portions of the sample 8 are sequentially irradiated with light, so that the sample 8 can be measured on a surface.

A procedure for analyzing the physical properties of the sample 8 in the present embodiment is basically the same as the procedure described in Embodiment 1 with reference to FIG. 2. The present embodiment is different in that after S6 of the flow shown in FIG. 2 is executed, the movable mirror 25 is driven and moved by one pitch to move an irradiation position of the light on the sample 8 by one pitch, and the steps from S1 are sequentially repeated. According to the present embodiment, since the position at which the sample 8 is irradiated with light is sequentially changed by the movable mirror 25, the sample 8 can be measured on the surface with a relatively simple configuration without using a driving mechanism for relatively moving the positions of the photo-acoustic cell 4 and the sample 8.

While the invention made by the inventor has been described in detail based on the embodiments, the invention is not limited to the embodiments described above, and various modifications can be made without departing from the scope of the invention. For example, the embodiments described above have been described in detail to facilitate understanding of the invention, and the invention is not necessarily limited to those including all the configurations described above. In addition, a part of the configuration of each embodiment may be added to, deleted from, or replaced with another configuration.

### Reference Signs List

1, 1A: light source
2: drive circuit
3: optical chopper
4: photo-acoustic cell
5: microphone
6: amplifier circuit
7: physical property analysis unit
8: sample
9: measurement area
10: speaker
11: signal generation circuit
12, 12-1: peak frequency search unit
14, 24: memory
15: frequency band switching unit
16: threshold determination unit
17: result display unit
19: frequency input unit
20: synchronization detection unit
21: camera
22: database
23: frequency characteristic measurement unit
25: movable mirror

## Claims

1. A photo-acoustic physical property measuring device that measures a physical property of an object by detecting a photo-acoustic signal generated by irradiating the object with light, the photo-acoustic physical property measuring device comprising:
a light irradiation unit configured to irradiate the object with modulated light;
a photo-acoustic cell configured to amplify a photo-acoustic wave generated at the object when the object is irradiated with the modulated light from the light irradiation unit;
a sound wave generation unit configured to generate a sound wave inside the photo-acoustic cell;
a microphone configured to detect a photo-acoustic wave amplified by the photo-acoustic cell and a sound wave generated inside the photo-acoustic cell from the sound wave generation unit; and
a physical property analysis unit configured to analyze the physical property of the object based on the photo-acoustic wave detected by the microphone.

2. The photo-acoustic physical property measuring device according to claim 1, wherein
the light irradiation unit further includes a light source, an optical chopper, a peak frequency search unit configured to detect a peak frequency of the sound wave that is generated inside the photo-acoustic cell from the sound wave generation unit and is detected by the microphone, and a drive circuit unit configured to drive the optical chopper based on the peak frequency of the sound wave generated inside the photo-acoustic cell from the sound wave generation unit that is searched for by the peak frequency search unit.

3. The photo-acoustic physical property measuring device according to claim 1, wherein
the sound wave generation unit includes a speaker configured to generate the sound wave inside the photo-acoustic cell, and a signal generation circuit unit configured to control a frequency band of the sound wave generated from the speaker.

4. The photo-acoustic physical property measuring device according to claim 2, wherein
the sound wave generation unit includes a speaker configured to generate the sound wave inside the photo-acoustic cell, a signal generation circuit unit configured to control a frequency band of the sound wave generated from the speaker, and a frequency band switching unit configured to control the signal generation circuit unit based on the peak frequency of the sound wave generated inside the photo-acoustic cell from the speaker that is detected by the peak frequency search unit and to switch the frequency band of the sound wave generated from the speaker.

5. The photo-acoustic physical property measuring device according to claim 1, wherein
the light irradiation unit further includes a light source, an optical chopper, a peak frequency search unit configured to detect a peak frequency of the sound wave that is generated inside the photo-acoustic cell from the sound wave generation unit and is detected by the microphone, a threshold determination unit configured to compare the peak frequency detected by the peak frequency search unit with a preset threshold, a display unit configured to display a result determined by the threshold determination unit, a frequency input unit configured to input a frequency for driving the optical chopper, and a drive circuit unit configured to drive the optical chopper based on the frequency for driving the optical chopper that is received from the frequency input unit.

6. The photo-acoustic physical property measuring device according to claim 1, wherein
the light irradiation unit further includes a light source, a peak frequency search unit configured to detect a peak frequency of the sound wave that is generated inside the photo-acoustic cell from the sound wave generation unit and is detected by the microphone, and a drive circuit unit configured to modulate and drive the light source based on the peak frequency of the sound wave generated inside the photo-acoustic cell from the sound wave generation unit that is searched for by the peak frequency search unit.

7. The photo-acoustic physical property measuring device according to claim 2, further comprising:
a synchronization detection unit configured to detect the photo-acoustic wave detected by the microphone in synchronization with the peak frequency of the sound wave generated inside the photo-acoustic cell from the sound wave generation unit that is detected by the peak frequency search unit, and send the signal detected in synchronization to the physical property analysis unit.

8. The photo-acoustic physical property measuring device according to claim 3, further comprising:
an imaging unit configured to image a positional relationship between the object and the photo-acoustic cell; a database configured to store a relationship between the positional relationship between the object and the photo-acoustic cell that is imaged by the imaging unit and a resonance frequency of the photo-acoustic cell, wherein the signal generation circuit unit controls the frequency band of the sound wave generated from the speaker based on the relationship between the positional relationship between the object and the photo-acoustic cell and the resonance frequency of the photo-acoustic cell stored in the database.

9. The photo-acoustic physical property measuring device according to claim 2, further comprising:
a frequency measurement unit configured to obtain a frequency characteristic from a signal obtained by detecting, by the microphone, the photo-acoustic wave generated in the photo-acoustic cell when the object is irradiated with the modulated light from the light irradiation unit; and a storage unit configured to store the frequency characteristic obtained by the frequency measurement unit, wherein the peak frequency search unit obtains the peak frequency of the sound wave generated inside the photo-acoustic cell using a signal obtained by detecting, by the microphone, the sound wave generated inside the photo-acoustic cell from the sound wave generation unit and the frequency characteristic stored in the storage unit, and controls the drive circuit unit based on the obtained peak frequency.

10. The photo-acoustic physical property measuring device according to claim 1, further comprising:
a movable mirror provided between the light irradiation unit and the photo-acoustic cell, wherein the movable mirror switches an optical path of the modulated light emitted from the light irradiation unit to change a position where the object is irradiated with the modulated light.

11. A photo-acoustic physical property measuring method comprising:
irradiating an object with light that is emitted from a light irradiation unit and is modulated at a predetermined frequency;
detecting, by a microphone, a photo-acoustic signal that is generated from the object irradiated with the light modulated at the predetermined frequency and resonates inside the photo-acoustic cell; and
analyzing a physical property of the object using the photo-acoustic signal detected by the microphone, wherein
the predetermined frequency of the light emitted from the light irradiation unit to the object is set based on a peak frequency searched for by a peak frequency search unit, and the peak frequency is obtained by
generating a sound wave inside the photo-acoustic cell from a speaker,
receiving, by the microphone, a sound generated inside the photo-acoustic cell by the generated sound wave, and
searching for, by the peak frequency search unit, a peak frequency of the sound received by the microphone.

12. The photo-acoustic physical property measuring method according to claim 11, wherein
emitting the light modulated at the predetermined frequency from the light irradiation unit is to cause the light emitted from the light source of the light irradiation unit to be modulated at the predetermined frequency by the optical chopper of the light irradiation unit and emit the light modulated at the predetermined frequency by the optical chopper.

13. The photo-acoustic physical property measuring method according to claim 12, wherein
modulating the light emitted from the light source at the predetermined frequency by the optical chopper is performed by detecting, by the peak frequency search unit, the peak frequency of the sound wave that is generated inside the photo-acoustic cell from the speaker and is detected by the microphone, and driving, by a drive circuit unit, the optical chopper based on the peak frequency of the sound wave generated inside the photo-acoustic cell from the speaker that is searched for by the peak frequency search unit.

14. The photo-acoustic physical property measuring method according to claim 11, wherein
a frequency band switching unit switches a frequency band of the sound wave generated inside the photo-acoustic cell from the speaker to sequentially generate sound waves having different frequency bands inside the photo-acoustic cell from the speaker, and the peak frequency search unit detects the peak frequency based on a signal obtained by detecting, by the microphone, the sequentially generated sound waves having different frequency bands.

15. A photo-acoustic physical property measuring method comprising:
irradiating an object with light that is emitted from a light irradiation unit and is modulated at a predetermined frequency;
detecting, by a microphone, a photo-acoustic signal that is generated from the object irradiated with the light modulated at the predetermined frequency and resonates inside the photo-acoustic cell; and
analyzing a physical property of the object using the photo-acoustic signal detected by the microphone, wherein
the predetermined frequency of the light emitted from the light irradiation unit to the object is set based on a peak frequency searched for by a peak frequency search unit, and the peak frequency is obtained by
sequentially irradiating the object with light having a different frequency to be modulated from the light irradiation unit, obtaining and storing a frequency characteristic of a photo-acoustic signal that resonates inside the photo-acoustic cell from a signal obtained by detecting, by the microphone, the photo-acoustic signal,
generating a sound wave inside the photo-acoustic cell from a speaker,
receiving, by the microphone, a photo-acoustic signal generated inside the photo-acoustic cell by the sound wave, and
searching for, by the peak frequency search unit, a peak frequency from a signal obtained by subtracting a component of the obtained and stored frequency characteristic from the photo-acoustic signal received by the microphone.
